# EUROPÄISCHE PATENTANMELDUNG

(11) **EP 0 948 969 A1**
(43) Veröffentlichungstag der Anmeldung: **13.10.1999**
(21) Anmeldenummer: 99105037.8
(22) Anmeldetag: 22.03.1999
(51) Int. Cl.: A61L 2/14

(54) **Verfahren zur Plasmabehandlung in Hohlkörpern**

(30) Priorität: 02.04.1998 DE 19814865
(71) Anmelder: FRAUNHOFER-GESELLSCHAFT ZUR FÖRDERUNG DER ANGEWANDTEN FORSCHUNG E.V., 80636 München (DE)
(72) Erfinder: Behnisch, Jürgen, 12209 Berlin (DE); Holländer, Andreas, 14513 Teltow (DE)
(74) Vertreter: Gagel, Roland, Dr.

(57) **Zusammenfassung**

Die vorliegende Erfindung betrifft ein Verfahren zur Plasmabehandlung in Hohlkörpern, insbesondere zur plasmachemischen Modifizierung von im Inneren von Hohlkörpern befindlichen Oberflächen. Bei dem Verfahren wird ein flexibler Hohlkörper bereitgestellt, der vor dem Verschließen unter Volumenkontraktion auf einen bestimmten Druck evakuiert wird. Dieser Druck wird so gewählt, daß der Hohlkörper unter reduziertem Außendruck in einer Plasmaprozeßkammer eine Volumenexpansion erfährt, bei der der Druck im Hohlkörper einen Wert erreicht, der bei Anlegen eines äußeren elektrischen Feldes eine Gasentladung im Hohlkörper zuläßt. Der auf diese Weise evakuiert und verschlossene Hohlkörper wird in eine Vakuumkammer gebracht, die derart evakuiert wird, daß sich im Hohlkörper die Voraussetzungen für das Zünden einer Gasentladung einstellen. Schließlich wird die Gasentladung innerhalb des Hohlkörpers durch Anlegen eines elektrischen Feldes gezündet.
Mit dem erfindungsgemäßen Verfahren läßt sich auf einfache und vorteilhafte Weise die Plasmabehandlung im Inneren von Hohlkörpern erreichen, ohne daß zusätzliche Modifikationen an bestehenden Plasmaanlagen erforderlich wären.

## Beschreibung

Die vorliegende Erfindung betrifft ein Verfahren zur Plasmabehandlung in Hohlkörpern, insbesondere zur plasmachemischen Modifizierung von im Inneren dieser Hohlkörper befindlichen Oberflächen.

Die Plasmabehandlung von Oberflächen spielt in vielen Bereichen der Technik eine große Rolle. So werden beispielsweise mittels plasmaunterstützter chemischer Dampfphasenabscheidung (PECVD) dünne Schichten aus den unterschiedlichsten Materialien auf zu bearbeitenden Oberflächen abgeschieden. Die abgeschiedenen Schichten können hierbei unterschiedliche Funktionen als Isolationsschicht, Leitfähigkeitsschicht, Diffusionsbarriere, usw. erfüllen.

Eine besondere Fallgruppe stellt die Plasmabehandlung im Inneren von Hohlkörpern dar. Beispielsweise kann die Plasmabehandlung der inneren Oberflächen von medizinischen Geräten, wie Kathetern, zur Verbesserung ihrer Biokompatibilität eingesetzt werden. Eine derartige Anwendung ist beispielsweise in der US 5 244 654 oder der US 5 486 357 beschrieben.

Ein weiteres Verfahren zur Plasmabehandlung ist in der US 5 700 327 beschrieben. Bei diesem Verfahren wird ein oxidierendes Prozeßgas in das Innere eines Containers geleitet, während in diesem ein niedriger Druck aufrechterhalten wird. Durch Anlegen eines elektrischen Feldes an den Container wird ein Plasma im Inneren gezündet, das eine Reinigungswirkung auf das Innere des Containers hat.

In der US 5 677 010 wird ein Verfahren beschrieben, bei dem im Inneren eines Kraftstofftanks eine Polymerschicht durch einen plasmachemischen Prozeß bei reduziertem Druck abgeschieden wird. Der Kraftstofftank befindet sich in einer Plasmaprozeßkammer, ist jedoch über eine separate gasdichte Leitung mit einer Vakuumpumpe außerhalb der Prozeßkammer verbunden. Der Druck innerhalb des Tanks wird über diese separate Leitung weiter abgesenkt als der Druck in der Prozeßkammer. Gleichzeitig wird ein Prozeßgas über eine weitere Leitung mit einer definierten Geschwindigkeit durch den Tank geleitet. Aufgrund der gewählten Druckverhältnisse wird nur innerhalb des Tanks ein Plasma gezündet.
Für die Durchführung des Verfahrens ist jedoch ein erheblicher apparativer Aufwand erforderlich, da das Innere des Tanks unabhängig vom Inneren der Vakuumkammer evakuiert und mit dem Prozeßgas versorgt werden muß. Dies kann nur über zusätzliche gasdichte Verbindungen von außerhalb der Vakuumkammer zum Inneren des Hohlkörpers erreicht werden.

Ein ähnliches Verfahren wird in der US 5 521 351 beschrieben, wobei in diesem Fall der Druck im Inneren des Hohlkörpers durch den dosierten Ein- und Auslaß eines Prozeßgases derart eingestellt wird, daß im Inneren des Hohlkörpers die Bedingungen für eine Gasentladung (Plasma) gegeben sind, während durch permanentes Abpumpen der Druck in der Prozeßkammer derart erniedrigt ist, daß dort keine Gasentladung möglich ist. Die plasmachemische Modifizierung findet somit nur im Inneren des Hohlkörpers statt. Auch hier ist der apparative Aufwand beträchtlich.

Eine spezielle Anwendung eines plasmachemischen Prozesses zur Sterilisierung der inneren Oberflächen von Plastikverpackungen ist in der US 5 531 060 beschrieben. Hierbei werden die Verpackungen durch Blasform-Technik hergestellt, bei der das Plastikmaterial unter Einwirkung eines entsprechenden Gasdruckes in eine vorbereitete Form gepreßt wird, die der Form der späteren Verpackung entspricht. In der Druckschrift wird vorgeschlagen, bei der Blasformung bereits ein für die spätere Plasmabehandlung geeignetes Gas zu verwenden und dieses durch nachfolgendes Verschließen der Verpackung darin einzuschließen. Die Verpackung wird dann in eine Vakuumkammer gebracht, an einer oder mehreren Stellen geöffnet und zusammen mit der Vakuumkammer evakuiert. Bei Erreichen eines geeigneten Druckes kann schließlich ein Plasma in der Verpackung gezündet werden.
Bei diesem Verfahren wird somit der Herstellungsprozeß bereits mit dem nachfolgenden Plasmabehandlungsprozeß kombiniert. Damit wird ein zusätzliches Zuführen des Prozeßgases in die Vakuumkammer nicht mehr erforderlich. Allerdings stößt das erforderliche Öffnen der Verpackung innerhalb der Vakuumkammer auf beträchtliche Probleme.

Die DE 44 08 301 beschreibt eine Vorrichtung zum Sterilisieren des Inneren eines Behälters, die mehrere Vakuumkammern aufweist. In einer der Vakuumkammern befinden sich Gasdüsen für den Einlaß eines Prozeßgases sowie Elektroden zum Zünden eines Niedertemperatur-Plasmas. Mit der Vorrichtung lassen sich geöffnete Hohlkörper im Innern sterilisieren. Hierzu wird eine der Elektroden vor dem Zünden des Plasmas in den Hohlkörper eingeführt. Die Vorrichtung mit drei hintereinander geschalteten Vakuumkammern ist jedoch sehr aufwendig.

Die DE 196 15 735 beschreibt eine Vorrichtung zum Sterilisieren der Innenflächen von druckempfindlichen Behältern mittels ionisierter Teilchen. Diese Vorrichtung besteht aus einer evakuierbaren Kammer zur Aufnahme der Behälter und einer an die Kammer angeschlossenen Zuleitung für zu ionisierendes Gas. In der Kammer befinden sich zwei einem Behälter zugeordnete Elektroden zum Erzeugen eines Plasmas. Die Vorrichtung zeichnet sich insbesondere dadurch aus, daß beide Elektroden außerhalb des zugehörigen Behälters diesen eng umschließend angeordnet sind. Hierdurch wird erreicht, daß innerhalb und außerhalb des Behälters der gleiche Unterdruck herrscht, und daß die zu sterilisierende Oberfläche zugleich im wesentlichen die plasmabegrenzende Wand ist, was zu einem guten Wirkungsgrad des Plasmas und zu einer kurzen Behandlungszeit führt. Die Elektrodenanordnung dieser Vorrichtung erfordert jedoch ganz bestimmte, je nach Größe der Behältnisse unterschiedliche, Ausgestaltungen der Prozeßkammer.

Die ältere, nachveröffentlichte DE 196 40 528 A1 beschreibt ein Verfahen zur vakuumtechnischen Behandlung von Teilen mit Niederdruckgasentladungen. Bei dem Verfahren werden die zu behandelnden Teile in vakuumtaugliche Verpackungsbehälter eingebracht. Die Behälter werden auf einen Druck evakuiert, der die Zündung einer Plasmaentladung durch Anlegen eines äußeren elektromagnetischen Wechselfeld zuläßt.

Aufgabe der vorliegenden Erfindung ist es, ein Verfahren zur Plasmabehandlung in Hohlkörpern bereitzustellen, das einfach und unter geringem apparativen Aufwand ohne Modifikationen an bestehenden Plasmaanlagen einsetzbar ist.

Die Erfindung wird mit dem Verfahren gemäß dem geltenden Anspruch 1 gelöst. Besondere Ausgestaltungen des Verfahrens sind Gegenstand der Unteransprüche. Des weiteren werden mit den Patentansprüchen 13 bis 18 Hohlkörper angegeben, die gemäß einem Aspekt der vorliegenden Erfindung ausgestaltet sind, so daß deren Inneres auf einfache Weise einer Plasmabehandlung unterzogen werden kann.

Erfindungsgemäß wird bei dem vorgeschlagenen Verfahren ein zumindest teilweise flexibler Hohlkörper bzw. ein Hohlkörper, der teilweise aus flexiblem Material besteht, eingesetzt. Der Hohlkörper wird über eine Öffnung evakuiert und im evakuierten Zustand verschlossen. Aufgrund der Flexibilität des Hohlkörpers tritt bei der Evakuierung eine Volumenkontraktion des Hohlkörperinnenraums auf. Der durch die Evakuierung im Innenraum des Hohlkörpers eingestellte Druck wird so gewählt, daß sich das Volumen des Hohlkörpers unter reduziertem Außendruck in einer Vakuumkammer wieder derart erweitert, daß der Druck im Hohlkörper beim Anlegen eines äußeren elektrischen Feldes eine nicht-thermische Gasentladung zuläßt.

Der verschlossene Hohlkörper wird schließlich in eine Vakuumkammer eingebracht. Die Vakuumkammer wird auf einen Druck evakuiert, bei dem der Druck im Hohlkörper die Zündung einer Gasentladung in dem Hohlkörper zuläßt. Bei einer derartigen Reduzierung des Druckes in der Vakuumkammer "bläht" sich der kontraktierte Hohlkörper wieder auf. Durch diese Volumenexpansion des Innenraums des Hohlkörpers erniedrigt sich der Druck im Hohlkörper bis schließlich die Bedingungen für die Zündung einer Gasentladung erreicht sind. Die Gasentladung wird in bekannter Weise durch Anlegen eines elektrischen Feldes an das den Hohlkörper beinhaltende Prozeßvolumen gezündet. Durch die Gasentladung wird ein Plasmaprozeß gestartet, mit dem die inneren Oberflächen des Hohlkörpers behandelt werden.

Als Hohlkörper können hierbei beispielsweise verschlossene Verpackungsbeutel aus Polymerfolien dienen, die von innen einschließlich möglicherweise darin verpackter Gegenstände an der Oberfläche modifiziert werden sollen.

Es versteht sich von selbst, daß die angegebenen Bedingungen bei der Evakuierung des Hohlkörpers nur eingehalten werden können, wenn eine ausreichende Volumenkontraktion des Hohlkörpers möglich ist, ohne diesen bei der Volumenexpansion zu zerstören.

Ebenso ist dem Fachmann geläufig, daß der Hohlkörper zumindest zum Teil aus dielektrischem Material bestehen muß, um eine Abschirmung des Innenraums des Hohlkörpers von dem angelegten elektrischen Feld zu vermeiden.

Mit dem erfindungsgemäßen Verfahren läßt sich in vorteilhafter und einfacher Weise eine Plasmabehandlung im Inneren von Hohlkörpern durchführen. Hierbei ist keine Modifikation bestehender Plasmaprozeßanlagen erforderlich. Des weiteren ist der apparative Aufwand zur Durchführung des Verfahrens gering.

Wie bei dem Verfahren der älteren, nachveröffentlichten DE 196 40 528 A1 wird beim vorliegenden Verfahren der Hohlkörper vor der Plasmabehandlung gasdicht verschlossen und bleibt während und unmittelbar nach der Behandlung in diesem verschlossenen Zustand.

Durch die Ausnutzung der Volumenkontraktion der flexiblen Hohlkörper bei der Evakuierung ist es nicht notwendig, ein derart starkes Vakuum in den Hohlkörpern zu erzeugen, wie dies für das Zünden eines Plasmas erforderlich wäre. Es reicht vielmehr ein höherer Druck aus, der beispielsweise mit einer einfachen Membranpumpe erzeugt werden kann. Die Hohlkörper können somit unabhängig von einer technologisch aufwendigen leistungsfähigen Vakuumpumpe, beispielsweise bereits an deren Herstellungsort, evakuiert und verschlossen werden. Der Plasmaprozeß kann schließlich in jeder kommerziellen Plasmaprozeßanlage ohne das Erfordernis zusätzlicher Modifikationen durchgeführt werden.

Bei dem erfindungsgemäßen Verfahren kann durch einfache Einstellung des Druckes in der Vakuumkammer bzw. Prozeßkammer eine plasmachemische Modifizierung entweder nur im Inneren des Hohlkörpers oder gleichzeitig im Inneren des Hohlkörpers und in der Prozeßkammer erfolgen. Somit kann beispielsweise nur die Innenseite des Hohlkörpers oder gleichzeitig die Innen- und Außenseite des Hohlkörpers modifiziert werden. Auch hierbei ist kein Umbau der gängigen Plasmaprozeßkammern notwendig.

Die Art des Prozeßgases zur Plasmabehandlung des Inneren des Hohlkörpers kann vor der Plasmabehandlung durch Spülen mit dem gewünschten Prozeßgas während des Evakuierens und Verschließens des Hohlkörpers festgelegt werden. Das Prozeßgas kann auch vor dem Evakuieren durch Zugabe einer flüssigen oder festen Substanz in den Hohlkörper eingebracht werden. Diese Substanzen werden durch die Evakuierung in den gasförmigen Zustand überführt.

Bei der gleichzeitigen Modifizierung der Innen- und Außenseite des Hohlkörpers können unterschiedliche Prozeßgase eingesetzt werden. Hierdurch kann beispielsweise die Außenseite des Hohlkörpers hydrophiliert werden (z.B. um eine gute Bedruckbarkeit zu erreichen), während gleichzeitig die Innenseite z.B. mit einer Barriereschicht versehen wird.

Bei Anwendungsfällen, bei denen ausschließlich die Oberflächen im Inneren des Hohlkörpers modifiziert werden, treten durch Einsatz des erfindungsgemäßen Verfahrens vorteilhafterweise keinerlei Kontaminationen in der Prozeßkammer und damit der gesamten Plasmaanlage einschließlich des Pumpsystems auf.

Des weiteren werden in diesem Fall keine besonderen Anforderungen an die Kontrolle des Vakuums in der Prozeßkammer gestellt, da der Prozeßdruck im Inneren des Hohlkörpers unterhalb eines bestimmten Druckes in der Prozeßkammer nicht mehr von diesem abhängt. Der Druck in der Prozeßkammer muß hierbei lediglich unterhalb des Druckes liegen, bei dem der Hohlkörper vollständig in seinem Volumen expandiert ist.

Mit dem erfindungsgemäßen Verfahren können in einer Prozeßkammer gleichzeitig in Material und Form unterschiedliche Hohlkörper modifiziert werden. Weiterhin können durch Vorsehen unterschiedlicher Prozeßgase in den verschiedenen Hohlkörpern gleichzeitig unterschiedliche Plasmaprozesse zur Modifikation eingesetzt werden. In gleicher Weise ist ein chargenweiser Wechsel des Prozeßgases im Inneren des Hohlkörpers möglich, ohne daß irgendwelche Veränderungen an der Plasmaanlage vorgenommen werden müssen.

Das erfindungsgemäße Verfahren bietet ganz besondere Vorteile in den Fällen, in denen das Ziel des Plasmaprozesses die Behandlung eines im flexiblen Hohlkörper (z.B. Folienbeutel) enthaltenen Gegenstandes ist. Der Gegenstand wird hierbei in dem bereits verschlossenen Hohlkörper der Plasmabehandlung unterzogen, so daß eine nachfolgende Verpackung entfällt. Hierdurch wird eine optimale Konditionierung im Vergleich zu einer separaten Plasmabehandlung mit anschließender Verpackung erreicht, bei der eine Kontamination des Gegenstandes durch die Umgebung nicht ausgeschlossen werden kann.

So können beispielsweise die bei der Sterilisation von Produkten im Bereich der Biologie und Medizin auftretenden Probleme gelöst werden. Insbesondere kann mit der Plasmabehandlung in der verschlossenen Verpackung eine Sterilisation ohne weiteren Kontakt mit der Außenwelt erreicht werden. Des weiteren kann die Plasmabehandlung beispielsweise mit dem Ziel einer verbesserten Benetzbarkeit oder einer besseren Biokompatibilität eines Produktes in der verschlossenen Verpackung gleichzeitig zu einer Sterilisation und damit zum bereits steril verpackten Endprodukt führen. Bei Einsatz des erfindungsgemäßen Verfahrens kann daher auf einen sehr aufwendigen separaten Sterilisationsprozeß verzichtet werden.

Weiterhin kann der Hohlkörper beim erfindungsgemäßen Verfahren auch als individuelle/separate Reaktionskammer für jeden zu behandelnden Gegenstand eingesetzt werden, die gegebenenfalls nach der Plasmabehandlung leicht entsorgt werden kann.

Die Erfindung wird nachfolgend anhand des grundsätzlichen Verfahrensablaufs und anhand von mehreren Ausführungsbeispielen nochmals erläutert.

Ein zumindest teilweise flexibler, wenigstens an einer Stelle noch offener Hohlkörper aus einem dielektrischen Material, beispielsweise ein Beutel aus einer Polymerfolie, wird nach einem der bekannten Verfahren durch Abpumpen unter teilweiser Volumenkontraktion evakuiert und vakuumdicht verschlossen. Dabei kann sich in diesem Hohlkörper ein Gegenstand befinden, beispielsweise eine Mikrotitterplatte aus Polystyren. Darüber hinaus kann der Hohlkörper während des Abpumpens mit einem speziellen Gas gespült werden, beispielsweise mit einem Inertgas wie Argon. Es kann jedoch auch vor dem Evakuieren eine flüssige Substanz, wie z.B. HMDSO, in den Hohlkörper gegeben werden. Der Hohlkörper muß außerdem nicht vollkommen aus dielektrischem Material bestehen. Wird auf das Spülen mit einem speziellen Prozeßgas verzichtet, so werden nach dem Evakuieren in normaler Umgebungsluft Sauerstoffmoleküle im Hohlkörper verbleiben, so daß das spätere Plasma eine oxidierende Wirkung auf die Oberflächen hat. Eine solche oxidierende Wirkung führt zur Sterilisierung der Oberflächen.

Das Volumen und die Geometrie des Hohlkörpers, gegebenenfalls unter Berücksichtigung eines darin eingeschlossenen Gegenstandes, sowie der Druck beim Verschließen des Hohlkörpers werden so gewählt, daß im weiteren Prozeßverlauf ein ausreichend freies Volumen und ein ausreichend niedriger Druck im Hohlkörper entstehen kann. Der ausreichend niedrige Druck von weniger als 10 mbar sowie das freie Volumen sind notwendig, um im Inneren des Hohlkörpers eine elektrische Gasentladung initiieren und aufrechterhalten zu können (Plasmaprozeß).

Je nach Art des Prozeßgases werden die Voraussetzungen für eine Zündung des Plasmas in der Regel in einem Druckbereich zwischen 0,1 und 1 mbar vorliegen. Bei bestimmten Prozeßgasen können die Druckwerte allerdings auch von diesem Bereich abweichen.

Der Hohlkörper wird schließlich in die Vakuumkammer einer Plasmaanlage gelegt, beispielsweise zwischen die beiden Elektroden in einer Parallelplattenanordnung. Selbstverständlich können auch gleichzeitig mehrere, möglicherweise auch unterschiedliche und mit unterschiedlichen Prozeßgasen gefüllte Hohlkörper in der Plasmaanlage bearbeitet werden.

Anschließend wird mit dem Abpumpen der Prozeßkammer begonnen. Wird der Innendruck des kontraktierten Hohlkörpers unterschritten, so beginnt sich das Volumen zu vergrößern (der Hohlkörper wird "aufgeblasen"). In dieser Phase des Abpumpprozesses entspricht der Druck in der Prozeßkammer dem Innendruck im Hohlkörper.

Bei weiterer Druckerniedrigung in der Prozeßkammer wird der Moment erreicht, in dem der Hohlkörper sein maximal mögliches Volumen einnimmt. Der Druck in der Prozeßkammer entspricht in diesem Moment dem niedrigsten zu erreichenden Innendruck im Hohlkörper, der sich ab diesem Zeitpunkt auch bei weiterer Druckerniedrigung in der Prozeßkammer nicht mehr verändert.

Bei Einkopplung eines elektrischen Feldes ausreichender Stärke in die Prozeßkammer wird ein Plasma an der Stelle gezündet, an der die für die Plasmazündung geeigneten Druckverhältnisse vorliegen. In Abhängigkeit von den Druckverhältnissen in der Prozeßkammer sind drei mögliche Prozeßregimes zu unterscheiden:
- das Plasma brennt nur in der Prozeßkammer außerhalb des Hohlkörpers;
- das Plasma brennt gleichzeitig in der Prozeßkammer (außerhalb des Hohlkörpers) und innerhalb des Hohlkörpers; und
- das Plasma brennt nur innerhalb des Hohlkörpers.

Alle drei Prozeßvarianten können durch entsprechende Einstellung der Druckverhältnisse realisiert werden.

Nach Beendigung der Plasmabehandlung durch Ausschalten des elektrischen Feldes können die Prozeßkammer belüftet und der Hohlkörper entnommen werden. Die Einkopplung des elektrischen Feldes in das Prozeßvolumen kann selbstverständlich mit Hilfe einer Vielzahl von Elektroden bzw. Antennenformen erfolgen, wie dies dem Fachmann auf diesem Gebiet bekannt ist. Die Einkopplung wird hierbei vorzugsweise mit Hochfrequenz oder Mikrowellenstrahlung realisiert. Geeignete Plasmaanlagen sind hinlänglich bekannt.

Das erfindungsgemäße Verfahren ist nicht auf eine bestimmte Art der Plasmabehandlung festgelegt. So können beliebige Schichten auf den inneren Oberflächen abgeschieden werden. Weiterhin kann die Behandlung der Oberflächen durch oxidierende Prozeßgase erfolgen. Grundsätzlich ist jede Form der Modifizierung von Oberflächen durch nicht-thermische Plasmen mit dem erfindungsgemäßen Verfahren durchführbar.

Anhand der folgenden drei Ausführungsbeispiele sollen zunächst die oben beschriebenen drei Prozeßregimes in der Prozeßkammer und deren Auswirkung veranschaulicht werden.

Zunächst wird ein flacher, an einer Stelle noch nicht verschlossener Beutel (10 x 10 cm²) aus Polyethylen mittels einer Membranpumpe auf einen Enddruck von ca.

10 mbar evakuiert und zugeschweißt. Dieser Beutel wird zwischen die Elektroden einer Plasmaanlage gelegt, die von einem HF-Generator bei 13,56 MHz gespeist wird.

Bei einer ersten Variante des Prozeßverlaufs wird die Prozeßkammer der Plasmaanlage auf einen Druck von 8 x 10⁻² mbar ausgepumpt. Die Druckverhältnisse im Beutel reichen bei diesem Außendruck zur Zündung eines Plasmas nicht aus. Durch Einschalten des HF-Generators (50 Watt) wird daher in der Prozeßkammer nur außerhalb des PE-Beutels für eine Minute ein Plasma gezündet. Im Ergebnis dieser Behandlung ändert sich der Kontaktwinkel mit Wasser an der Außenseite des PE-Beutels zu 52° gegenüber 95° vor der Behandlung, während er an der Innenseite des Beutels unverändert 95° beträgt. Dies konnte durch Messungen festgestellt werden.

Gemäß einer zweiten Prozeßvariante wird die Prozeßkammer diesmal auf einen Druck von 3,5 x 10⁻² mbar ausgepumpt. Bei diesem Druck sind die Bedingungen für eine Plasmazündung sowohl innerhalb des Beutels als auch in der Prozeßkammer erfüllt. Durch Einschalten des HF-Generators (50 Watt) wird in der Prozeßkammer daher gleichzeitig außerhalb des PE-Beutels und innerhalb des PE-Beutels für eine Minute ein Plasma gezündet. Im Ergebnis dieser Behandlung wurde der Kontaktwinkel mit Wasser an der Außenseite des PE-Beutels zu 52° und an der Innenseite Beutels zu 48° gegenüber jeweils 95° vor der Behandlung gemessen.

Gemäß der dritten Variante wird die Prozeßkammer auf einen Druck von 1 x 10⁻² mbar ausgepumpt. Dadurch sind die Bedingungen für eine Plasmazündung nur innerhalb des Beutels erfüllt. Durch Einschalten des HF-Generators (50 Watt) wird bei diesen Verhältnissen daher nur innerhalb des PE-Beutels für eine Minute ein Plasma gezündet, während außerhalb des PE-Beutels kein Plasma entsteht. Im Ergebnis dieser Behandlung wurde der Kontaktwinkel mit Wasser an der Außenseite des PE-Beutels zu 95° und an der Innenseite des Beutels zu 49° gegenüber jeweils 95° vor der Behandlung gemessen.

Bei einem weiteren Ausführungsbeispiel wird ein flacher, an einer Stelle noch nicht verschlossener Beutel (10 x 10 cm²) aus Polyethylenterephthalat (PETP) mittels einer Membranpumpe auf einen Enddruck von ca. 10 mbar evakuiert, mit einem Prozeßgas (Hexafluorethan, C₂F₆) gespült und zugeschweißt. Dieser Beutel wird zwischen die Elektroden einer Plasmaanlage gelegt, die von einem HF-Generator mit 13,56 MHz gespeist wird. Die Prozeßkammer der Plasmaanlage wird abgepumpt und ein zweites Prozeßgas (Hexamethyldisiloxan, HMDSO) wird mit einem Fluß von 20 sccm in die Prozeßkammer eingeleitet. Der resultierende Druck in der Prozeßkammer beträgt 3 x 10⁻² mbar. Durch Einschalten des HF-Generators (50 Watt) werden in der Prozeßkammer außerhalb des PE-Beutels für 10 Minuten ein HMDSO-Plasma und gleichzeitig innerhalb des Beutels ein C₂F₆-Plasma gezündet. Im Ergebnis dieser Behandlung wird auf der Außenseite des PETP-Beutels, wie durch XPS-Messungen nachgewiesen werden konnte, eine siliziumorganische Plasmapolymerschicht abgeschieden, während auf der Innenseite des PETP-Beutels ein Wasserkontaktwinkel von 110° gegenüber 63° vor der Behandlung gemessen werden konnte. Darüber hinaus wurde bei XPS-Messungen an der Innenseite nach der Behandlung Fluor nachgewiesen.

Bei einem weiteren Ausführungsbeispiel wird eine flache, an einer Stelle noch nicht verschlossene Tüte (10 x 15 cm²), wie sie zur Sterilverpackung benutzt wird, mit einer darin befindlichen Mikrotitterplatte (4 x 4 x 1 cm³) aus Polystyren (PS) mittels einer Membranpumpe auf einen Enddruck von 10 mbar evakuiert und versiegelt. Dieser versiegelte Beutel wird in eine Plasmaanlage gelegt, die mit einem Mikrowellengenerator bei 2,54 GHz gespeist wird. Die Prozeßkammer der Plasmaanlage wird auf einen Druck von 1 x 10⁻² mbar ausgepumpt. Durch Einschalten des Mikrowellengenerators (250 Watt) wird in der Prozeßkammer innerhalb des PE-Beutels für 5 Minuten ein Plasma gezündet, während außerhalb kein Plasma gezündet wird. Durch diese Behandlung wird eine Verbesserung der Benetzbarkeit bei gleichzeitiger Sterilisierung der Mikrotitterplatte erzeugt. Wird der geschlossene Beutel 14 Tage nach der Behandlung geöffnet, so ist im Ergebnis selbst nach dieser Zeit die Mikrotitterplatte noch vollständig mit Wasser benetzbar und biologisch steril.

## Patentansprüche

1. Verfahren zur Plasmabehandlung in Hohlkörpern mit folgenden Verfahrensschritten:
- Bereitstellen eines eine Öffnung aufweisenden Hohlkörpers, der zumindest teilweise flexibel ausgestaltet ist;
- Evakuieren des Hohlkörpers unter Volumenkontraktion auf einen ersten Druck, der so gewählt ist, daß der evakuierte und verschlossene Hohlkörper in der Umgebung eines zweiten Druckes eine Volumenexpansion erfährt, durch die der Druck im Hohlkörper einen Wert annimmt, der die Zündung einer nicht-thermischen Gasentladung im Hohlkörper zuläßt;
- gasdichtes Verschließen des Hohlkörpers unter Beibehaltung des ersten Druckes;
- Einbringen des Hohlkörpers in eine Vakuumkammer;
- Evakuieren der Vakuumkammer auf den zweiten Druck; und
- Zünden einer Gasentladung im verschlossenen Hohlkörper durch Anlegen eines elektrischen Feldes.

2. Verfahren nach Anspruch 1, dadurch gekennzeichnet, daß der zweite Druck so eingestellt wird, daß in der Vakuumkammer bei Anlegen des elektrischen Feldes auch außerhalb des verschlossenen Hohlkörpers eine Gasentladung gezündet wird.

3. Verfahren nach Anspruch 1, dadurch gekennzeichnet, daß der zweite Druck so eingestellt wird, daß außerhalb des verschlossenen Hohlkörpers bei Anlegen des elektrischen Feldes keine Gasentladung gezündet wird.

4. Verfahren nach einem der Ansprüche 1 bis 3, dadurch gekennzeichnet, daß der Hohlkörper vor oder während des Evakuierens mit einem ersten Prozeßgas gespült wird.

5. Verfahren nach einem der Ansprüche 1 bis 3, dadurch gekennzeichnet, daß vor dem Evakuieren eine feste oder flüssige Substanz in den Hohlkörper eingebracht wird, die ein erstes Prozeßgas bildet.

6. Verfahren nach einem der Ansprüche 4 oder 5, dadurch gekennzeichnet, daß in die Vakuumkammer ein zweites Prozeßgas eingeleitet wird, das dem ersten Prozeßgas entspricht oder eine andere Zusammensetzung als das erste Prozeßgas aufweist.

7. Verfahren nach einem der Ansprüche 1 bis 6, dadurch gekennzeichnet, daß vor dem Verschließen des Hohlkörpers ein Gegenstand, der einer Plasmabehandlung unterzogen werden soll, in den Hohlkörper eingebracht wird.

8. Verfahren nach Anspruch 7 zur Sterilisation von Produkten.

9. Verfahren nach Anspruch 7 zur Plasmabehandlung von Gegenständen, bei dem der Hohlkörper als individuelle Reaktionskammer für den Gegenstand dient, die nach der Plasmabehandlung leicht entsorgt werden kann.

10. Verfahren nach einem der Ansprüche 1 bis 9, dadurch gekennzeichnet, daß es sich bei dem Hohlkörper um Verpackungsmaterial handelt.

11. Verfahren nach einem der Ansprüche 1 bis 10, dadurch gekennzeichnet, daß das Verschließen durch Verschweißen erfolgt.

12. Verfahren nach einem der Ansprüche 1 bis 11, bei dem gleichzeitig mehrere evakuierte und verschlossene Hohlkörper in die Vakuumkammer eingebracht werden, wobei bei Anlegen des elektrischen Feldes in jedem der Hohlkörper eine Gasentladung gezündet wird.

13. Hohlkörper, der zumindest teilweise flexibel ausgestaltet und allseitig gasdicht verschlossen ist, wobei der Hohlkörper unter Volumenkontraktion auf einen ersten Druck evakuiert ist, der so gewählt ist, daß der evakuierte und verschlossene Hohlkörper in der Umgebung eines zweiten Druckes eine Volumenexpansion erfährt, durch die der Druck im Hohlkörper einen Wert annimmt, der die Zündung einer nicht-thermischen Gasentladung im Hohlkörper zuläßt.

14. Hohlkörper nach Anspruch 13, der einen Gegenstand beinhaltet.

15. Hohlkörper nach Anspruch 13 oder 14, der ein Gas beinhaltet, das im Plasmazustand einen sterilisierende Wirkung auf angrenzende Oberflächen zeigt.

16. Hohlkörper nach Anspruch 13 oder 14, der ein Gas beinhaltet, das im Plasmazustand eine diffusionshemmende Schicht auf angrenzende Oberflächen abscheidet.

17. Hohlkörper nach Anspruch 13 oder 14, der ein Gas beinhaltet, das im Plasmazustand eine erhöhte Benetzbarkeit von angrenzenden Oberflächen bewirkt.

18. Hohlkörper nach Anspruch 13 oder 14, der ein Gas beinhaltet, das im Plasmazustand eine verbesserte Biokompatibilität von angrenzenden Oberflächen bewirkt.
